(19) **Europäisches Patentamt**

**European Patent Office**

**Office européen des brevets**

(11) **EP 1 008 330 A2**

(12) **EUROPEAN PATENT APPLICATION**

(43) Date of publication:
**14.06.2000 Bulletin 2000/24**

(51) Int. Cl.⁷: **A61F 13/02**

(21) Application number: **99124010.2**

(22) Date of filing: **08.12.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **08.12.1998 JP 34919798**

(71) Applicant:
**Johnson & Johnson Kabushiki Kaisha
Tokyo 135 (JP)**

(72) Inventor: **Yoshida, Takeshi
Fukushima-ken (JP)**

(74) Representative:
**Groening, Hans Wilhelm, Dipl.-Ing.
BOEHMERT & BOEHMERT
Franz-Joseph-Strasse 38
80801 München (DE)**

(54) **Adhesive bandage**

(57)   An adhesive bandage (1) having a backing sheet (2) and an adhesive layer (14) on one side of said backing sheet (2) characterized in that: said adhesive bandage (1) is comprised of a first adhesive portion (5) which is adhered to a finger-cushion surface of fingertip, and a second adhesive portion (7) which is folded from said finger-cushion side to be adhered to a nail-side surface of the fingertip so as to face the first adhesive portion (5) across the fingertip; said first adhesive portion (5) has first projecting portions (5a) which are folded from the finger-cushion side and adhered to each side surface of the fingertip; said second adhesive portion (7) has second projecting portions (7a) and third projecting portions (7b) folded from the nail-side toward the finger-cushion side and wound around the fingertip; and said fingertip is enveloped in the first adhesive portion (5) and the second adhesive portion (7).

This adhesive bandage (1) can firmly envelop an affected part of a fingertip of hand or foot, and prevent intrusion of water, bacteria, etc. through a gap which may conventionally be formed during application or decrease in adhesion properties between the pad and the affected part if an adhesive bandage having a pad is applied.

FIG. 1

**EP 1 008 330 A2**

**Description**

This invention relates to a bandage which firmly envelops an affected part on a fingertip of a hand or foot so that invasion of water, bacteria, etc. can be prevented, and further an adhesive bandage which is formed by setting a pad on the bandage.

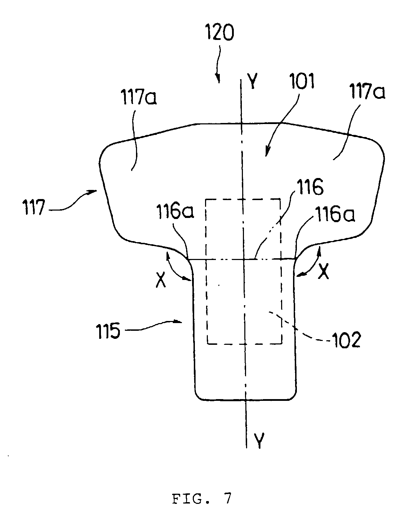

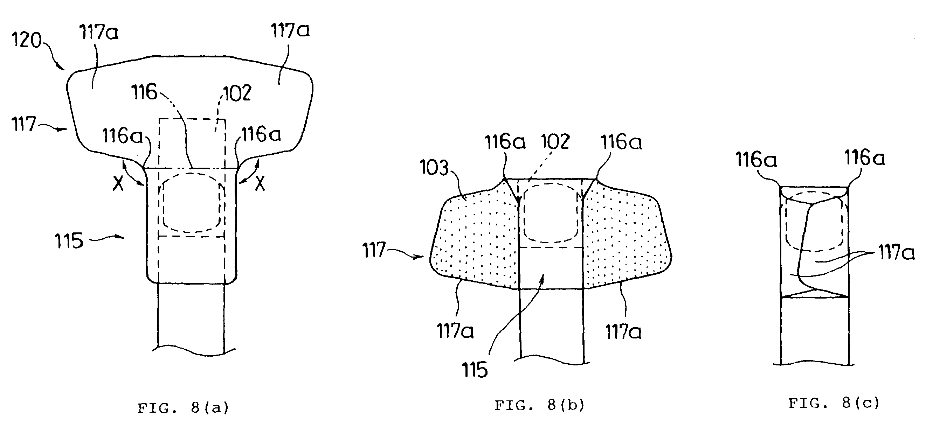

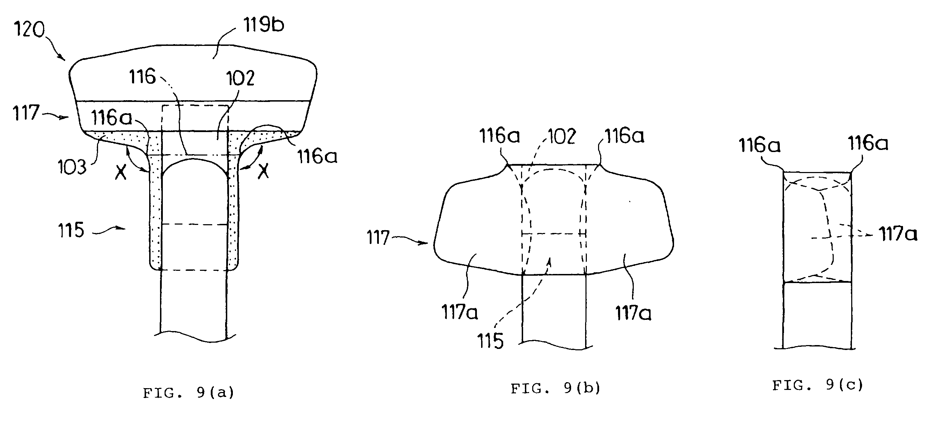

There is an adhesive bandage in the form shown in Fig. 7 as a conventional adhesive bandage for application to a fingertip of hand or foot. Fig. 7 is a plane view of the conventional adhesive bandage 120 for application to a fingertip. Figs. 8(a) to (c) and Figs. 9(a) to (c) are drawings for illustrating a method of applying the conventional adhesive bandage 120 to a fingertip of hand, Fig. 8 is plane views viewing from a nail-side of the fingertip, and Fig. 9, viewing from a finger-cushion side. The conventional adhesive bandage 120 will be explained hereafter while referring to Figs. 7 to 9. In Figs. 8 and 9, a fiber fabric 103 is shown in a way of pointillism for the sake of illustration.

As shown in Fig. 7, conventional adhesive bandage 120 for use in fingertip is formed in an approximate-T shape as a whole, and which is made up of a backing sheet consisting of a fiber fabric 103 (see Fig. 8 and Fig. 9) and a film 101 laminating said fiber fabric 103, an adhesive layer (not shown) formed on a surface of the backing sheet on which said fiber fabric 103 is set, and a pad 102 set on said adhesive layer (not shown) which is attached to an affected part. Said adhesive bandage 120 may have release papers covering an adhesive surface, a backing sheet surface of adhesive-layer side (not shown). With regard to said release papers, only a release paper 119b covering second adhesive portion 117 is shown in Fig. 9(a).

Said adhesive bandage 120 has a first adhesive portion 115 adhered to the nail-side of a finger and a second adhesive portion 117 adhered to the finger-cushion side opposite to said first adhesive portion by being folded from the nail-side of the finger, and the adhesive bandage can be folded at the folding area 116 which is a boundary between said first adhesive portion 115 and said adhesive portion 117. The pad 102 lies across the folding area 116. The adhesive bandage 120 is shaped symmetrically against the hypothetical center line Y-Y in a direction perpendicular to the long direction of the folding area 116.

Said first adhesive portion 115 is formed in a rectangular shape having a long side in a direction toward the fingertip adhered, that is, a direction perpendicular to the folding area 116. And said second adhesive portion 117 is approximate-rectangular shape having its long side in the direction parallel to the folding area 116, projecting by the same lengths from the hypothetical center line Y-Y to both directions parallel to said folding area 116, and the portions longer than the folding area form the projection portions 117a.

An angle X formed between the first adhesive portion 115 and the second adhesive portion 117 outside the adhesive bandage 120 is obtuse angle.

Then, an application method of the conventional adhesive bandage is explained.

(a) Firstly, as shown in Fig. 8(a) and Fig. 9(a), peeling off the release paper (not shown) covering the adhesive portion 115, and adhering said first adhesive portion 115 to the nail-side surface of the fingertip.

(b) As shown in Fig. 8(b) and Fig. 9(b), peeling off the release paper 119b, folding said second adhesive portion 117 at the folding area from the nail-side, and adhering it to the finger-cushion side of the finger in the state opposite to the first adhesive portion 115.

(c) As shown in Fig. 8(c) and Fig. 9(c), turning back the second projecting portion 117a from said finger-cushion side toward said nail-side, and winding up around the fingertip each second projecting portion 117a projecting in each direction parallel to said folding area 116, that is, lateral directions of the fingertip.

By steps of above (a) to (c), the conventional adhesive bandage 120 is attached to the affected part of the fingertip of hand or foot.

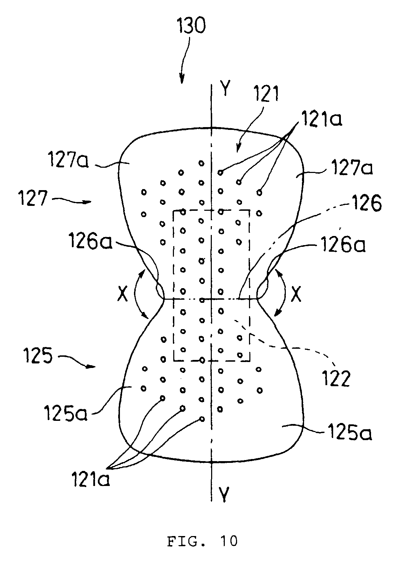

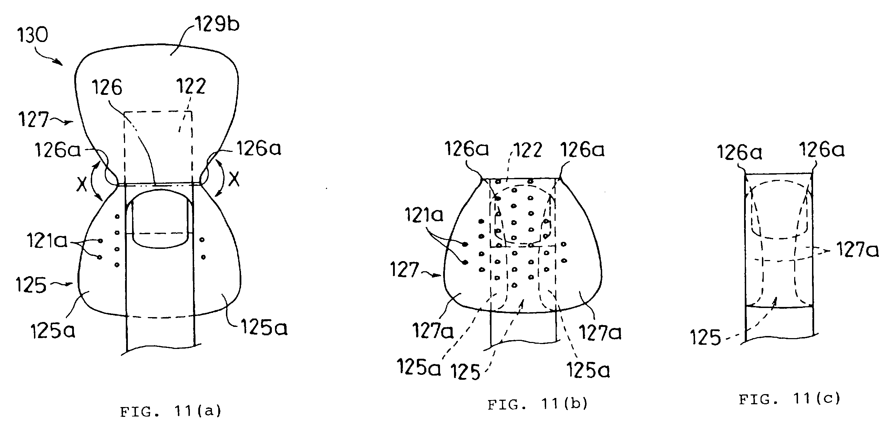

Then, another conventional adhesive bandage 130 for use in fingertip of hand or foot will be explained while referring to Fig. 10 and Fig. 11. Fig. 10 shows a plane view of the conventional adhesive bandage 130, and Figs. 11(a) to (c) show a method of adhering the conventional adhesive bandage 130 to a fingertip, which are plane views viewing from nail side.

As shown in Fig. 10, the conventional adhesive bandage 130 is formed in approximate-8-figure shape as a whole and comprised of a film 121 as a backing sheet, an adhesive layer (not shown) formed on a side of said backing sheet and a pad 122 set on said adhesive layer (not shown) which is attached to an affected part. The adhesive bandage 130 has a release paper covering the adhesive layer surface, that is, a backing sheet surface of the adhesive-layer side, and said film 12 1 has air holes 121a. As to the release paper, only a release paper 129b is shown in Fig. 11(a) covering the second adhesive portion 127 described below.

Said adhesive bandage 130 has a first adhesive portion 125 adhered to a finger cushion of a fingertip of hand or foot, for example, and a second adhesive portion 127 folded from the finger-cushion side and adhered to the nail-side of the fingertip opposite to said first adhesive portion 125, and can be folded at the folding area 126, which is a boundary between the first adhesive portion 125 and the second adhesive portion 127. The first adhesive portion 125 and the second adhesive portion 127 is formed in the same shape with each other, and the shape of the adhesive bandage 130 is symmetric with respect to the folding area 126 and to the hypothetical center line Y-Y perpendicular to the folding area 126. The pad 122 also lies across the folding area 126.

The first adhesive portion 125 and the second adhesive portion 127 are approximate-trapeziforms of which peripheries are formed of a curved lines, and said folding area 126 corresponding to an upper side is a short side. That is, a long side corresponding to a lower side projects from the hypothetical center line Y-Y toward the both directions parallel to the folding area 126 by the same lengths. The wider portions than the folding area form the first projecting portions 125a and the second projecting portions 127a.

Then, an application method of the conventional adhesive bandage 130 to a fingertip of hand will be explained.

(A) Firstly peeling the release paper (not shown) covering the first adhesive portion 125 as shown in Fig. 11(a), adhering the first adhesive portion 125 to the finger cushion, folding the first projecting portion 125a from the finger-cushion side and adhering to side surfaces of the fingertip.

(B) As shown in Fig. 11(b), peeling the release paper 129b, folding the second adhesive portion 127 from the finger-cushion side at the folding area 126, and adhering to the nail-side of the fingertip opposite to the first adhesive portion 125.

(C) As shown in Fig. 11(c), the second projecting portions 127a projecting to the directions parallel to the folding area 126 are folded from the nail-side toward the finger-cushion side and adhered to the side surfaces of the fingertip.

By the steps (A) to (C) above, the conventional adhesive bandage 130 is adhered to the fingertip so that the pad 122 is attached to an affected part of the fingertip of hand or foot. In this case, because the first adhesive portion 125 and the second adhesive portion 127 is in the approximate trapezoidal shape with curved peripheral and formed large comparing to the fingertip, the adhesive bandage 130 is easy to conform to the shape of the fingertip and to envelop the fingertip.

The conventional adhesive bandages 120 and 130, however, are required improvements regarding following points.

Since the first adhesive portion 115 and the second adhesive portion 117 of the conventional adhesive bandage 120 are in the approximate-rectangular shape respectively, the angle X formed between the first adhesive portion 115 and the second adhesive portion 117 outside the adhesive bandage 120 is large, and when adhered to a fingertip, there is a problem such that a clearance gap is apt to be formed near edge portion 116a of the folding area 116. Thus a problem will arise such that water gets in from the clearance gap so that the pad gets wet during such as bathing or washing.

Further, since the first adhesive portion 115 and the second adhesive portion 117 are approximate-rectangular shapes, the conventional adhesive bandage 120 is difficult to fit to the curved surface of the fingertip and apt to decrease adherence between the pad 102 and the affected part.

And in the case of the conventional adhesive bandage 130, since the first adhesive portion 125 and the second adhesive portion 127 are respectively formed in trapezoidal shapes, the angle X which is formed by peripheral curves of the first adhesive portion 125 and the second adhesive portion outside the adhesive bandage 130 becomes comparatively large. Therefore when it is adhered to a fingertip, a clearance gap is apt to be formed near edge portion 126a of the folding area 126, and water intrudes from the clearance gap so that the pad 122 gets wet with the water.

This invention aims to solve above-said problems and to provide bandage which tightly envelops an affected part of a fingertip of hand or foot to prevent intrusion of such as water and bacteria, in particular an adhesive bandage formed of the bandage and a pad.

An adhesive bandage of the present invention is an adhesive bandage having a backing sheet and an adhesive layer on one side of said backing sheet characterized in that: said adhesive bandage is comprised of a first adhesive portion which is adhered to a finger-cushion surface of fingertip, and a second adhesive portion which is folded from said finger-coshion side to be adhered to a nail-side surface of the fingertip so as to face the first adhesive portion across the fingertip; said first adhesive portion has first projecting portions which are folded from the finger-cushion side and adhered to each side surface of the fingertip; said second adhesive portion has second projecting portions and third projecting portions folded from the nail-side toward the finger-cushion side and wound around the fingertip; and said fingertip is enveloped in the first adhesive portion and the second adhesive portion.

The adhesive bandage of the present invention can be that having folding area on the border between said first adhesive portion and said second adhesive portion, and a shape of said adhesive bandage is symmetrical with respect to a hypothetical center line in the direction perpendicular to said folding area.

The second projecting portions and the projecting portions of the adhesive bandage of the present invention can be constructed to extend in the direction parallel to the folding area, and second projecting portions and said first adhesive portion adjoin across said folding area.

The second projecting portions and the third projecting portions of the adhesive bandage of the present invention can be placed separately in the direction perpendicular to said folding area, said second projecting portion euch has a straight line approximately parallel to said folding area, and said third projecting portion has approximately rectangular shape.

The length of said first projecting portion in the direction parallel to said folding area of the adhesive bandage according to the present invention can be shorter than tbat of the second projecting portion and the third projecting portion, and the periphery of the first projecting portion is formed of curved-line.

The length of said third projecting portion in the direction parallel to the folding area of the adhesive bandage according to the present invention can be longer than that of said second projecting portion.

The adhesive bandage of the present invention may have a pad lying across the folding area.

The adhesive bandage according to the present invention may have release papers covering the surface of said adhesive layer.

A form of working examples of this invention will be explained hereafter while referring to drawings showing in:

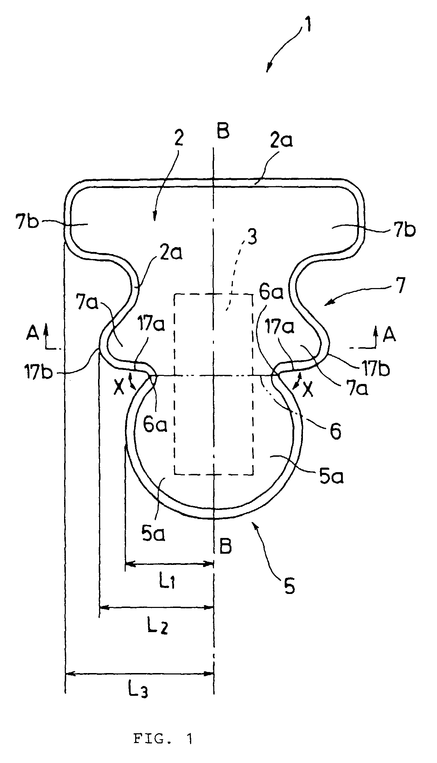

Fig. 1 a plan view of the adhesive bandage 1 of the present invention;

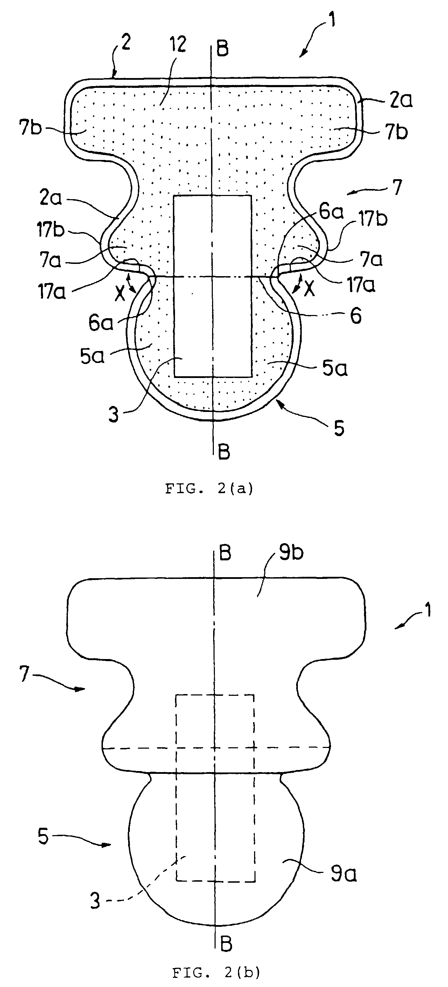

Fig. 2 (a) a plan view of the adhesive-side surface of the adhesive bandage 1 of the present invention, and
Fig. 2 (b) a plan view of the state the release paper covers the adhesive surface in above (a);

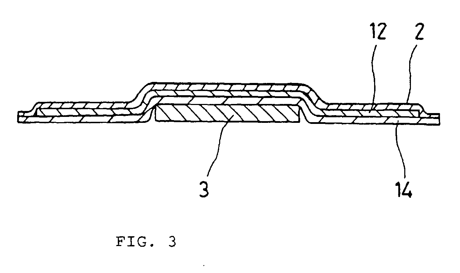

Fig. 3 a schematic drawing showing A-A section in Fig. 1;

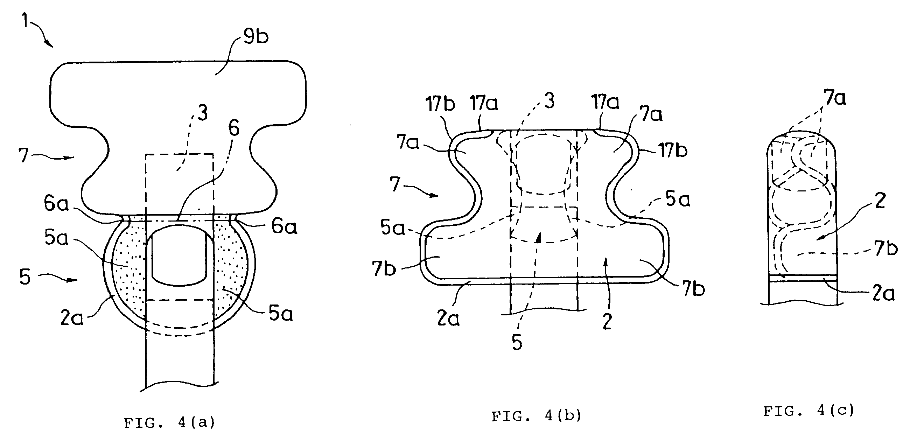

Fig. 4 (a) to (c) plan views showing a method of applying the adhesive bandage 1 of the present invention viewing from nail-side of the fingertip;

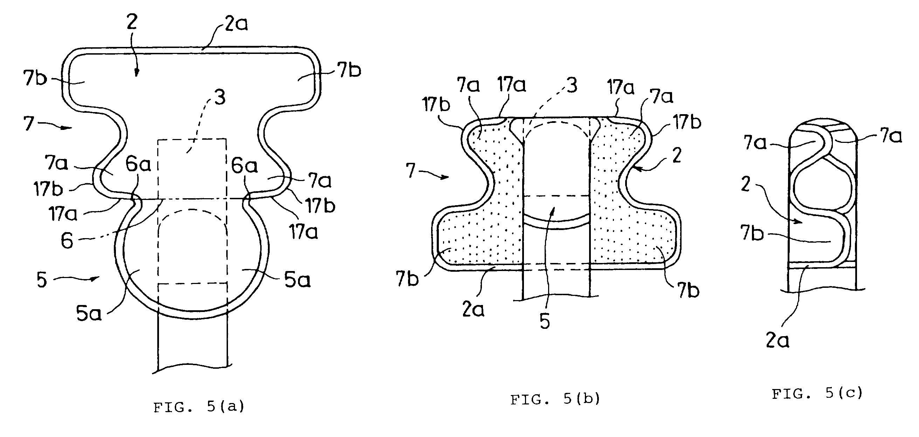

Fig. 5 (a) to (c) plan views showing a method of applying the adhesive bandage 1 of the present invention viewing from finger-cushion side of the fingertip;

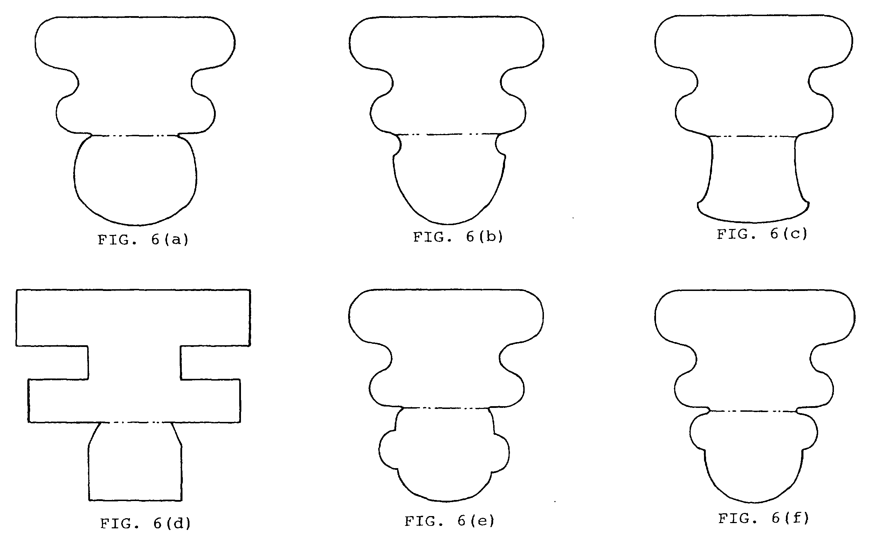

Fig. 6 (a) to (f) plan views illustrating the other Examples of forms of the adhesive bandage of the present invention;
Fig. 7 a plan view of a conventional adhesive bandage 120 for fingertip;
Fig. 8 (a) to (c) plan views showing a method of applying the conventional adhesive bandage to a fingertip of hand viewing from nail-side of the fingertip;
Fig. 9 (a) to (c) plan views showing a method of applying the conventional adhesive bandage viewing from finger-cushion side of the fingertip;
Fig. 10 a plan view showing a conventional adhesive bandage 130; and
Fig. 11 (a) to (c) plan views showing a method of applying the conventional adhesive bandage 130 viewing from nail-side of the fingertip.

Fig. 1 is a plan view of an adhesive bandage of the present invention. Fig. 2 (a) is a plan view illustrating an adhesive-layer side of the adhesive bandage of this invention. Fig. 2 (b) is a plan view illustrating a state covering the adhesive-layer surface with a release paper in Fig. 2 (a). Fig. 3 illustrates schematically A-A section of the Fig. 1. The adhesive bandage 1 of this invention will be explained referring Fig. 1 to 3.

As shown in Fig. 1 to 3, the adhesive bandage 1 is composed of a backing sheet comprising a thermoplastic fiber fabric 12 (shown in Fig. 2(a)) and a film 2 laminating the thermoplastic fiber fabric 12, an adhesive layer 14 (illustrated in Fig. 3) formed on the thermoplastic-fiber-fabric 12 side surface of the backing sheet, and a pad 3 set on the adhesive layer surface 14 and which will be attached to an affected part. And the adhesive bandage has a release paper 9a and a release paper 9b covering the adhesive surface, that is, the adhesive-layer-side surface of the backing sheet. Said release paper 9a is set so as to cover the first adhesive portion 5 described below, and said release paper 9b, the second adhesive portion described below. In the Fig. 2(a), said thermoplastic fiber fabric 12 is illustrated in a way of pointillism for illustration purpose.

The adhesive bandage 1 described above has a first adhesive portion 5 which is adhered to finger cushion of a fingertip of hand or foot and a second adhesive portion 7 which is folded from said finger-cushion side, adhered to a nail-side surface of the fingertip so as to face the first adhesive portion 5 across the fingertip, and said first adhesive portion 5 and said second adhesive portion 7 may be folded at the folding area 6. That is, said first adhesive portion 5 and said second adhesive portion 7 are integrally formed and it is folded at the folding area and adhered to the fingertip. Said pad 3 is lying across the folding area and the adhesive bandage 1 is formed in a shape symmetrical with respect to a hypothetical center line B-B in the direction perpendicular to the folding area.

The periphery of the first adhesive portion 5 is formed in approximate circular curve, and has the first projecting portions 5a projecting toward the both directions parallel to the folding area from said hypothetical center line B-B. The maximum radius, the length of said first projecting portion 5a from the hypothetical center line B-B, is L1.

The second adhesive portion 7 is formed projecting toward the both directions parallel to the folding area 6 from said hypothetical center line B-B, and has second projecting portions 7a and third projecting portions 7b separately arranged in the direction perpendicular to said folding area By forming the second projecting portions, the adhesive surface of the second projecting portion and that of corresponding first projecting portion intervened by the gap X can be bonded to each other when this adhesive bandage is applied so that intrusion of water may be completely avoided.

The periphery of the third projecting portion 7b toward 2a overlaps the periphery of the first projecting portion 5a in about the constant width so that intrusion of water may be avoided.

Said second projecting portions 7a adjoin the first adhesive portion 5 across the folding area 6, project from said hypothetical center line B-B by the length of L2 respectively, have an straight-line section 17a nearly parallel to the folding area 6, and periphery of the tip area 17b is curved The third projecting portions 7b project from said hypothetical center line B-B by the length of L3 respectively, have a rectangular-like shape periphery. That is, two third projecting portions must have enough length so that the tips of the projecting portion may wind around the finger from respective directions and may overlap with each other when this adhesive bandage is applied, and thereby intrusion of water from the palm direction may be avoided.

The gap X formed by the first adhesive portion 5 and the second projecting portion 7 outside the adhesive bandage 1 is narrow at acute angle because the periphery of the first adhesive portion 5 is curved and has the first projecting portion and the second projecting portion 7 has a straight-line section 17a approximately parallel to the folding area 6.

The lengths of the first projecting portion 5a, the second projection portion 7a and the third projecting portion 7b from the hypothetical center line B-B, L1, L2 and L3 respectively, have the relation shown by the following formula (1):

$$L3 > L2 > L1. \qquad (1)$$

That is, in the lengths of the first projecting portion 5a, the second projection portion 7a and the third projecting portion 7b in the direction parallel to the folding area, the length of the third projecting portion 7b is the largest of all, then the second projecting portion 7a and then the first projecting portion 5a.

The materials of respective parts of the adhesive bandage 1 will be explained as follows:

The thermoplastic fiber fabric 12 preferably has a high gas permeability and elasticity and includes woven fabrics and nonwoven fabrics made of thermoplastic elastomers. The thermoplastic elastomer can be, for example, polystyrene-type elastomers such as styrene-isoprene-styrene type block copolymer and hydrogenated block copolymers hydrogenating said block copolymer, polyurethanes, polyesters and polyolefines such as polyethylene, they are, however, not critical and other material may be selected. The fabric of the present invention can be either a woven fabric or a nonwoven fabric, but the nonwoven fabric is preferred because the directional dependency of physical properties such as elasticity is low.

When using a nonwoven fabric as the thermoplastic fiber fabric 12, the percentage expansion of the nonwoven fabric is preferably 100% or more, a recovery rate in expansion is 70 % or more. The weight of the nonwoven fabric of this invention has to be properly selected depending on properties of the nonwoven fabric itself, the laminating film and the adhesive, but cannot particularly be limited. However, the weight is preferably 20 to 200 g/m², more preferably 30 to 100 g/m². A thickness of the nonwoven fabric may be such that a sufficient stiffness is imparted to the backing sheet of the adhesive bandage when laminating a film on the nonwoven fabric. The thickness of the nonwoven fabric is about 20 to 1,000 μm, preferably 50 to 500 μm.

The film 2 is used to impart water proofing property to the adhesive bandage 1 without much decrease in gas permeability and water vapor permeability of the thermoplastic fiber fabric 12 as well as to ensure a suitable balance of properties when an adhesive bandage is made. The material of the film is required to have a water proofing property and to have sufficient water vapor permeability even after laminated on a thermoplastic fiber fabric 12. That is, the water vapor can permeate the film and liquid water cannot permeate, and as such films, known films made of polyurethanes, polyvinyl chloride, polyvinylidene chloride, polyolefines such as polyethylene and polypropylene, polyesters, polyamides and so forth can be used. Polyurethane films and polyester films in particular are preferred among them because they have high water vapor permeability and appropriate flexitility.

As above-said polyester elastomers, for example, polyester elastomer "Hytrel" (Trademark of E. I. DU PONT DE NEMOURS AND COMPANY), "Fleclone"(Trademark of Nichigo Film Kabushiki Kaisha) and so forth are preferred. Since the laminating film 2 must have a sufficient water proofing property, a film made by extrusion molding, blow-molding or the like is desirous. A drawn film is also available. Said laminating film can be also a multilayered film formed by laminating films made of different materials.

If the material of the laminating film has a low water vapor permeability, the film 2 has to be thin in order to have a high water vapor permeability. If the material of the laminating film has high water vapor permeability, it can be thick, but has to have suitable stiffness wLen laminated on the fiber fabric. From this standpoint, the thickness of said film is preferably 50 μm or less, more preferably 2 to 30 μm, and further more preferably 5 to 15 μm.

The adhesive used in the adhesive bandage of the present invention is not particularly limited as far as the skin is little irritated and pressure-sensitive adhesion to skin is provided. Examples of the adhesive include rubbery adhesives, acrylic adhesives, polyurethane adhesives, silicone adhesives and A-B-A type block copolymer type adhesives such as styrene-isoprene-styrene block copolymer type adhesives, etc.

The thickness of the adhesive layer 14 is 25 to 150μm, preferably 30 to 60 μm. The adhesive layer 14 can be formed by flat coating in which the adhesive is coated on the whole surface of the backing sheet composed of the film 2 and the thermoplastic fiber fabric 12, however coating a porous adhesive or pattern coating is preferred in order to ensure high water vapor permeability of the adhesive layer.

As a method of making the adhesive porous, there is, for example, a method in which a highly water-absorbable polymer is used as a blowing agent, which fully absorbs water, the resulting polymer is dispersed into an adhesive solution, the dispersion is coated, and a moisture is then evaporated to make the adhesive porous. This method is, however, not critical. Regarding the pattern coat, the adhesive can be coated on the backing sheet by, for example, screen coating or gravure coating. As a method of coating the adhesive on the backing sheet, there can be taken various known methods such as a method in which the backing sheet is directly coated with the adhesive, a method in which a release paper is coated with the adhesive and then the adhesive is transferred onto the backing sheet, and the like.

In this Example, the film 2 and thermoplastic fiber fabric 12 are laminated in the state all the periphery is heat-sealed in a constant width, in this case, about 1.5 mm width. The laminating method is not particularly restricted, and a method of heat-melting, a method using an adhesive can be adopted. The laminate can be performed either before forming the adhesive layer 14 or after forming the adhesive layer 14 on the backing sheet.

The heat-sealed section 2a formed by heat-sealing near the periphery of the film 2 and the thermoplastic fiber fabric 12 is formed on the whole periphery thereof and has a certain width, in this Example, about 1.5mm. Although the width of the heat-sealed section 2a is not particularly limited, 0.5~4mm is preferred, and 1~2mm is particularly preferred.

Methods of heat-seal can be that of pressing a subject sheet with a flat face maintaining a predetermined temperature, tbat of pressing a sheet with such as a rotary press, etc., the method is, however, not critical. The flat seal can be used for the heat-seal, and also the pattern seal can be adopted. The pattern seal refers to a heat-seal method tbat does not seal the whole surface of the sealing area but unsealed surface is remained in the sealing area. The pattern seal provides the sealing area 2a with a higher gas permeability and a water vapor permeability since the unsealed surface has higher gas permeability and water vapor permeability than the sealed surface.

The peripheral end of the backing sheet constructed by laminating the thermoplastic fiber fabric 12 and the film 2 is sealed by the heat-scaled section 2a, and the outside environment and the pad 3 are perfectly isolated. Therefore the adhesive bandage 1 can prevent intrusion of water from outside.

When using a material of the film 2 having higher melting point than the thermoplastic fiber fabric 12 and setting the heat-sealing temperature lower than the melting point of the film 2 and higher than the melting point of the thermoplastic fiber fabric, only the fiber fabric melts and the heat-sealed section can be formed in the same process as the cutting process of the backing sheet. In such process, the film 2 and the thermoplastic fiber fabric 12 is formed in the same size, and positioning between the film 2 and the thermoplastic fiber fabric 12 is avoided. For example, in the case of the combination of polyurethane film (melting point about 160 to 200 °C) or polyester (the melting point about 170 to 210 °C) as the film 2 and hydrogenated styrene-isoprene-styrene block copolymer (the melting point about 120 °C), the condition is satisfied. Thus only the fiber fabric 12 melts when heat-sealing is performed at temperatures near the melting point of the film.

Referring now to Fig. 4 and Fig. 5, an application method of the adhesive bandage of this invention to a fingertip of a hand or foot will be explained.

(a) to (c) of Fig. 4 and (a) to (c) of Fig. 5 are figures showing a method of applying the adhesive bandage of the present invention. Fig. 4 includes plane views viewing from nail-side of the fingertip and Fig. 5 includes plane views viewing from finger-cushion-side of the fingertip. In Figs. 4 and 5, the thermoplastic fiber fabric is illustrated in a way of pointillism for illustration purpose.

(1) As shown in Fig. 4(a) and Fig. 5(a), at first the release paper 9a (see Fig. 2(b)) is peeled, the first adhesive portion 5 is adhered to the finger cushion of the fingertip, the first projecting portion 5a is folded from the finger-cushion side and adhered to a side of the fingertip. In this case, it is preferred to adjust the fingertip at the center of the pad.

(2) As shown in Fig. 4(b) and Fig. 5(b), the release paper 9b is peeled, the second adhesive portion 7 is folded from the finger-cushion side and adhered to the nail side of the fingertip opposing the first adhesive portion.

(3) As shown in Fig. 4(c) and Fig. 5(c), adhesive layer end of the second projecting portion 7a projecting toward both directions parallel to the folding area 6 and opposing adhesive layer end of the first adhesive portion are adhered to each other, and respectively folded from the nail side to the finger-cushion side and wound around the fingertip. In this case, it is preferred to apply it to the fingertip while pulling lightly the second projecting portion 7a.

(4) As similar to above (3), the third projecting portion 7b in the state projecting in both directions parallel to the folding area 6, that is side directions of the fingertip, is folded from the nail side to the finger-cushion side. And periphery of 2a side of the third projecting portion 7b overlaps the periphery of the first projecting portion 5a at a constant width. In this case, it is preferred to apply it to the fingertip while pulling lightly the third projecting portion 7b.

By the processes (1) to (4) above, The adhesive bandage 1 is adhered to a fingertip of a hand or foot. In this case, the gap X formed by the first adhesive portion 5 of which periphery is formed of curved line and the second adhesive portion 7 having the straight line section 17a approximately parallel to the folding area 6 outside the adhesive bandage 1 is small. When the adhesive bandage 1 is applied to a fingertip, the gap is hardly formed near the end section 6a of the folding area 6. That is, the adhesive bandage 1 is seal from the outside environment by envelopping the fingertip with the first adhesive portion 5 and second adhesive portion 7, thereby intrusion of water, bacteria, etc. is prevented.

In the adhesive bandage 1, since the first adhesive portion 5 and the second adhesive portion 7 are formed large comparing to a fingertip, respective lengths in the direction parallel to the folding area 6 are arranged in order of the third projecting portion 7b the longest, the second projecting portion 7a, and the first projecting portion 5a the shortest, it is easy to fit with curved surface of fingertip and can firmly envelop the fingertip.

Further since the pad 3 lies across the folding area, the pad 3 can be firmly attachod to an affected part of the fingertip enveloped by the first adhesive portion 5 and the second adhesive portion 7.

Although this Example illustrates the adhesive bandage 1 having the pad 3, this invention can be applied to to these Examples.

The shapes of adhesive bandages of the present invention are not limited to these Examples, and can be, for example, Fig. 6(a) to (f). In Fig. 6(a) to (f), each folding area which is a boundary between the first adhesive portion and the second adhesive portion is shown by two dotted dashed line. The Fig. (e) to (f) is drawings having projecting portion further projecting from the first projecting portion of the first adhesive portion. Further the other shapes can be adopted to the present invention as far as satisfying essential conditions of the present invention.

Further forms of the present invention can be as follows:
The backing sheet is composed of thermoplastic fiber fabric and thermoplastic film; the adhesive bandage can be formed of thermoplastic fiber fabric; the thermoplastic fiber fabric is styrene-isoprene-styrene type block copolymer; the thermoplastic film is polyurethane film; the adhesive is porous; the whole periphery of the backing sheet is sealed.

As described above, the adhesive bandage of the present invention can firmly envelop an affected part of a fingertip of hand or foot, and prevent intrusion of water, bacteria, etc. through a gap which may be formed during application or decrease in adhesion properties between the pad and the affected part if an adhesive bandage having a pad is applied.

Reference Numerals

| 1 | adhesive bandage |
| 2 | film (backing sheet) |
| 2a. | heat-sealed section |
| 3 | pad |
| 5 | first adhesive portion |
| 5a | first projecting portion |
| 6 | folding area |
| 6 | end portion |
| 7 | second adhesive portion |
| 7a | second projecting portion |
| 7b | third projecting portion |
| 9a,9b | release papers |
| 14 | adhesive layer |
| 17a | straight line section (second projecting portion), |
| 17b | end section (second projecting portion) |

## Claims

1. An adhesive bandage (1) having a backing sheet (2) and an adhesive layer (14) on one side of said backing sheet (2)
characterized in that:

  said adhesive bandage (1) is comprised of a first adhesive portion (5) which is adhered to a finger-cushion surface of fingertip, and a second adhesive portion (7) which is folded from said finger-cushion side to be adhered to a nail-side surface of the fingertip so as to face the first adhesive portion (5) across the fingertip;
  said first adhesive portion (5) has first projecting portions (5a) which are folded from the finger-cushion side and adhered to each side surface of the fingertip;
  said second adhesive portion (7) has second projecting portions (7a) and third projecting portions (7b) folded from the nail-side toward the finger-cushion side and wound around the fingertip; and
  said fingertip is enveloped in the first adhesive portion (5) and the second adhesive portion (7).

2. The adhesive bandage according to claim 1 which has a folding area (6) on the border between the first adhesive portion (5) and the second adhesive portion (7), and a shape of said adhesive bandage (1) is symmetrical with respect to a hypothetical center line in the direction perpendicular to said folding area (6).

**3.** The adhesive bandage according to Claim 2 wherein the second projecting portions (7a) and the third projecting portions (7b) are constructed to extend in the direction parallel to said folding area (6), and the second projecting portions (7b) adjoin said first adhesive portion (5) over the folding area (6).

**4.** The adhesive bandage according to Claim 2 or 3 wherein said second projecting portions (7a) and said third projecting portions (7b) are allocated separately in the direction perpendicular to said folding area (6), each second projecting portion (7a) has a straight-line section (17a) approximately parallel to said folding area (6), and a shape of the third projecting portion (7b) is approximately rectangular.

**5.** The adhesive bandage according to any one of Claims 2 to 4 wherein the length of said first projecting portion (5a) in the direction parallel to said folding area (6) is shorter than those of the second projecting portion (7a) and the third projecting portion (7b), and the periphery of said first projecting portion (5a) is formed of curved-line.

**6.** The adhesive bandage according to any one of Claims 2 to 5 wherein the length of said third projecting portion (7b) in the direction parallel to the folding area (6) is longer than that of said second projecting portion (7a).

**7.** The adhesive bandage according to any one of Claims 2 to 6 wherein said adhesive bandage (1) has a pad (3) lying across the folding area (6).

**8.** The adhesive bandage according to any one of Claims 1 to 7 which has release papers covering the surface of the adhesive layer (14).

**9.** The adhesive bandage according to claims 1 to 8 wherein the backing sheet is composed of thermoplastic fiber fabric and thermoplastic film.

**10.** The adhesive bandage according to claim 9 formed of thermoplastic fiber fabric.

**11.** The adhesive bandage according to claim 10 wherein the thermoplastic fiber fabric is styrene-isoprene-styrene type block copolymer.

**12.** The adhesive bandage according to any one of claims 9 to 11 wherein the thermoplastic film is polyurethane film.

**13.** The adhesive bandage according to any one of claims 1 to 12 wherein the adhesive is porous.

**14.** The adhesive bandage according to any one of claims 9 to 13 wherein the whole periphery of the backing sheet is sealed.

FIG. 1

FIG. 2(a)

FIG. 2(b)

FIG. 3

FIG. 4(a)          FIG. 4(b)          FIG. 4(c)

EP 1 008 330 A2

FIG. 5 (a)

FIG. 5 (b)

FIG. 5 (c)

FIG. 6(a)

FIG. 6(b)

FIG. 6(c)

FIG. 6(d)

FIG. 6(e)

FIG. 6(f)

FIG. 7

FIG. 8(a)

FIG. 8(b)

FIG. 8(c)

FIG. 9 (c)

FIG. 9 (b)

FIG. 9 (a)

17

FIG. 10

FIG. 11(c)

FIG. 11(b)

FIG. 11(a)